# EUROPÄISCHE PATENTANMELDUNG

(11) **EP 2 730 259 A1**
(43) Veröffentlichungstag der Anmeldung: **14.05.2014**
(21) Anmeldenummer: 13005273.1
(22) Anmeldetag: 08.11.2013
(51) Int. Cl.: A61F 5/56

(54) **Okklusionsschiene**

(30) Priorität: 09.11.2012 DE 102012021895; 18.03.2013 DE 102013004517
(71) Anmelder: Bruderhofer, Andreas, 81379 München (DE); Kursawe, Leon, 81379 München (DE)
(72) Erfinder: Bruderhofer, Andreas, 81379 München (DE); Kursawe, Leon, 81379 München (DE)
(74) Vertreter: Heuer, Wilhelm

(57) **Zusammenfassung**

Eine Okklusionsschiene umfasst einen linken und einen rechten Distanzkörper (3), die geformt sind, um wenigstens einen linken bzw. einen rechten Backenzahn (7) eines Patienten zu überdecken, und ein sich bogenförmig zwischen dem linken und dem rechten Distanzkörper (3) erstreckendes Mittelstück (6). Das Mittelstück (6) berührt lingual Schneidezähne (5) des Patienten, lässt aber deren Inzisalkante (18) unbedeckt.

## Beschreibung

Okklusionsschienen werden in Zahnheilkunde und Kieferorthopädie zur Korrektur von Fehlstellungen der Zähne und des Kiefergelenks, zur Verhinderung bzw. Therapie von unbewusstem nächtlichem Zähneknirschen, Schnarchen und dergleichen eingesetzt. Sie umfassen im Allgemeinen einen einteiligen Kunststoffkörper, der sich U-förmig über Backen- und Schneidezähne eines Kiefers erstreckt. Konturen an Ober- und Unterseite des Kunststoffkörpers sind an die Oberflächenkonturen der Zähne von Unter- und Oberkiefer angepasst, um einerseits die Okklusionsschiene an einem Kiefer fest verrasten zu können und andererseits, wenn die Okklusionsschiene zwischen Zähnen des Ober- und Unterkiefers geklemmt ist, eine gewünschte Stellung des Kiefergelenks zu erzielen und/oder eine die Zahnstellung korrigierende Kraft auf einen oder mehrere Zähne oder eine sonstige korrigierende Wirkung auszuüben.

Wenn eine Okklusionsschiene getragen wird, ist ein direkter Kontakt der Zähne von Ober- und Unterkiefer miteinander in der Regel nicht mehr möglich. Ein Zahn, der keinem Druck eines gegenüberliegenden Zahnes ausgesetzt ist, neigt dazu, solange aus dem Kiefer herauszuwachsen, bis er auf einen Widerstand am gegenüberliegenden Kiefer trifft. Damit das Tragen einer Okklusionsschiene nicht dazu führt, dass einzelne Zähne ihr Widerlager verlieren, dadurch aus dem Kiefer herauswachsen und letztlich locker werden, sind Okklusionsschienen herkömmlicherweise so geformt, dass sie die Kronen sämtlicher Zähne des Kiefers, an dem sie befestigt sind, überdecken und in einer geschlossenen Stellung der Kiefer den von den Zähnen des Unterkiefers ausgeübten Druck auf sämtliche Zähne des Oberkiefers verteilen. So liefert die Okklusionsschiene den Druckreiz, den die Zähne benötigen, um nicht aus dem Kiefer herauszuwachsen.

Zur Bildung zahlreicher Laute der menschlichen Sprache ist es notwendig, dass die Zungenspitze die Schneidezähne berührt bzw. über Gaumenfreiheit verfügt. Eine Okklusionsschiene, die einen solchen Kontakt verhindert, indem sie die Kronen der Schneidezähne überdeckt bzw. die Bewegungsfreiheit der Zungenspitze einschränkt, stellt ein erhebliches Hindernis für eine klare Aussprache dar. Viele Patienten, insbesondere solche, die beruflich auf die klare Verständlichkeit ihrer Sprache angewiesen sind, können eine Okklusionsschiene daher nur nachts tragen, was Behandlungen mit Okklusionsschienen langwierig macht und die therapeutische Prognose einschränkt.

Aufgabe der Erfindung ist, eine Okklusionsschiene zu schaffen, die, indem sie die Artikulationsfähigkeit ihres Trägers nicht beeinträchtigt, auch tagsüber getragen werden kann und mit der infolgedessen erheblich kürzere Behandlungszeiten bei besserer therapeutischer Prognose erreichbar sind.

Die Aufgabe wird gelöst, indem bei einer Okklusionsschiene mit einem linken und einem rechten Distanzkörper, die geformt sind, um wenigstens einen linken bzw. einen rechten Backenzahn eines Patienten zu überdecken, und einem sich bogenförmig zwischen dem linken und dem rechten Distanzkörper erstreckenden Mittelstück das Mittelstück Schneidezähne des Patienten lingual berührt und deren Inzisalkante unbedeckt lässt. Es hat sich nämlich herausgestellt, dass der von einem solchen schmalen Mittelstück ausgehende Berührungsreiz ausreicht, um ein Herausschieben des Schneidezahns aus dem Kiefer zu verhindern. Die Inzisalkante kann daher frei bleiben, wodurch die Irritation der Zunge und die Beeinträchtigung der Artikulationsfähigkeit beim Tragen der erfindungsgemäßen Okkusionsschiene wesentlich geringer ist als bei einer herkömmlichen, die Inzisalkante überdeckenden Okklusionsschiene. Daher kann die erfindungsgemäße Okklusionsschiene auch tagsüber getragen werden, so dass die Dauer einer Okklusionsschienenbehandlung entsprechend verkürzt werden kann.

Vorzugsweise bleibt nicht nur die Inzisalkante, sondern auch ein daran angrenzender Bereich der lingualen Flanken der Schneidezähne unbedeckt.

Damit die Zungenspitze die Inzisalkante gut erreichen kann, sollte der unbedeckte Bereich der lingualen Flanken wenigstens ein Viertel, vorzugsweise wenigstens ein Drittel der Flanken einnehmen.

Um dem Mittelstück den für eine ausreichende Stabilität der Okklusionsschiene erforderlichen Querschnitt geben zu können, ohne es gleichzeitig so dick machen zu müssen, dass es den Träger irritiert, kann das Mittelstück in apikaler Richtung so verbreitert sein, dass es auch an die Schneidezähne angrenzendes Zahnfleisch überdeckt.

Die Okklusionsschiene kann mehrteilig ausgebildet sein, mit einem Bügel, von dem distale Bereiche formschlüssig in den Distanzkörpern eingebettet sind und ein zentraler Bereich wenigstens einen Teil des Mittelstücks bildet. Während die Distanzkörper vorzugsweise aus Kunstharz gefertigt sind, um sie effizient passend zu den Zähnen des Patienten formen zu können, kann der Bügel aus belastbarerem Material, insbesondere aus Metall, gefertigt sein. Dies erlaubt es, den Querschnitt des Mittelstücks sehr klein zu halten, was wiederum dazu beiträgt, die Behinderung der Zungenbewegung durch die Okklusionsschiene zu minimieren.

Um eine feste, auch gegen Torsionsmomente hinreichend beständige Verankerung des Bügels in den Distanzkörpern zu gewährleisten, weisen die distalen Bereiche des Bügels vorzugsweise ein höheres Querschnitts-Aspektverhältnis auf als dessen zentraler Bereich.

Einer Ausgestaltung zufolge können der linke und der rechte Distanzkörper ausschließlich durch den Bügel verbunden sein, d.h. der Bügel bildet das gesamte Mittelstück.

Denkbar ist aber auch, dass wenigstens ein Teil des Mittelstücks mit dem linken und dem rechten Distanzkörper einteilig zusammenhängt.

In diesem Fall verläuft der Bügel vorzugsweise beabstandet von den Schneidezähnen, und der Kontakt mit den Schneidezähnen wird hergestellt durch den mit den Distanzkörpern zusammenhängenden Teil des Mittelstücks, der im Allgemeinen aus einem leichter bearbeitbaren Material als der -vorzugsweise metallische- Bügel besteht.

Wenigstens das mechanisch relativ hoch belastete Mittelstück kann eine Einlage aus Fasern, insbesondere Glas-, Kohlenstoff- oder Aramidfasern aufweisen. Eine solche Einlage kann den metallischen Bügel ersetzen.

Die gesamte Okklusionsschiene kann einheitlich aus faserverstärktem Kunststoff gefertigt sein. Falls nicht die gesamte Okklusionsschiene faserverstärkt ist, sollten eine Matrix, in die die Fasern eingebettet sind, und ein faserfreier Bereich der Kaukörper aus gleichen oder zumindest kompatiblen Kunststoffen bestehen, um eine innige, feste Verbindung zwischen faserhaltigen und faserfreien Bereichen der Okklusionsschiene zu erhalten.

Eine jeweils am vorderen Ende der Distanzkörper geformte Erhebung kann genutzt werden, um die Position des Unterkiefers in der Schließstellung festzulegen bzw., wenn gewünscht, zu korrigieren.

Die Erhebungen haben zweckmäßigerweise eine erste Flanke, die in Schließstellung des Unterkiefers die palatale Flanke eines Eckzahns des Oberkiefers berührt. Kommt diese Flanke beim Schließen des Unterkiefers in Kontakt mit dem Eckzahn, bevor die Backenzähne des Oberkiefers die Distanzkörper berühren, dann wird der Unterkiefer beim Schließen zurückgeschoben. Eine Asymmetrie dieser Flanken zwischen den zwei Distanzkörpern kann eine Seitwärtsauslenkung des Unterkiefers bewirken. Eine weitere Flanke sollte beim Schließen des Unterkiefers in Kontakt mit einem ersten Prämolar kommen. Dieser Kontakt kann den Unterkiefer beim Schließen nach vorn ziehen.

Weitere Merkmale und Vorteile der Erfindung ergeben sich aus der nachfolgenden Beschreibung von Ausführungsbeispielen unter Bezugnahme auf die beigefügten Figuren. Es zeigen:
- Fig. 1: einen Unterkiefer mit einer erfindungsgemäßen Okklusionsschiene;
- Fig. 2: einen Distanzkörper im Querschnitt
- Fig. 3: einen Schnitt entlang der Mesialebene der Okklusionsschiene;
- Fig. 4: eine Okklusionsschiene mit einem verstärkenden Metallbügel;
- Fig. 5: eine Okklusionsschiene mit zwei voneinander getrennten Distanzkörpern; und
- Fig. 6: eine Okklusionsschiene mit faserverstärktem Mittelstück.

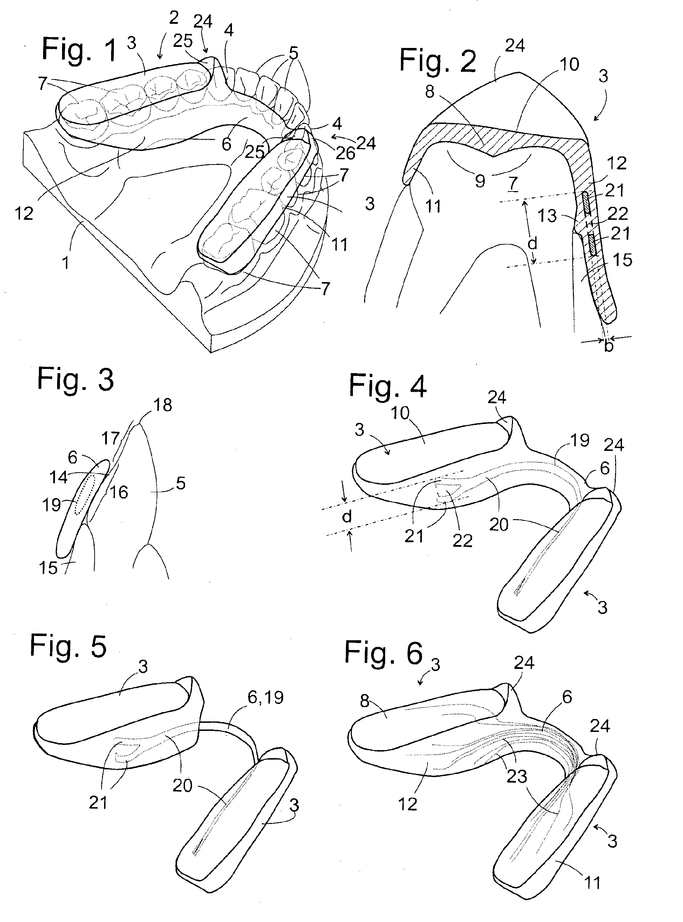

Fig. 1 zeigt in einer perspektivischen Ansicht einen Unterkieferabguss 1 eines Patienten mit einer darauf montierten Okklusionsschiene 2. Die Okklusionsschiene 2 ist im einfachsten Fall ein einteiliger Körper aus homogenem Kunststoff mit zwei seitlichen Distanzkörpern 3 und einem die Distanzkörper 3 verbindenden, sich entlang der lingualen Flanken von Eckzähnen 4 und Schneidezähnen 5 bogenförmig erstreckenden Mittelstück 6. Die Distanzkörper 3 überdecken alle vorhandenen Backenzähne 7, hier je vier Stück auf der linken und der rechten Seite, oder zumindest diejenigen Backenzähne 7, die ein Gegenstück im Oberkiefer haben.

Fig. 2 zeigt einen Distanzkörper 3 im Querschnitt. Eine im Wesentlichen horizontale obere Platte 8 des Distanzkörpers 3 liegt auf den Kauflächen der Backenzähne 7 auf und ist an ihrer Unterseite mit die Höcker der Backenzähne 7 formschlüssig aufnehmenden Einbuchtungen 9 versehen. Die Oberseite der Platte 8 ist zum großen Teil durch eine im Wesentlichen ebene Fläche 10 gebildet; zur Kauflächenkontur der Backenzähne des Oberkiefers komplementäre Einbuchtungen sind nicht vorhanden. Lediglich an den mesialen Enden der Distanzkörper 3 ist jeweils eine pyramiden- oder tetraederähnliche Erhebung 24 geformt, die über die ebene Fläche 10 aufragt.
Seitliche Schenkel 11, 12 der Distanzkörper 3 liegen an den Backzähnen 7 bukkal bzw. lingual an, um einen festen Halt der Schiene 2 auch bei geöffnetem Mund zu gewährleisten. Der linguale Schenkel 12 erstreckt sich bis auf das apikal an den Backenzahn 7 angrenzende Zahnfleisch 15. In Höhe des Zahnhalses kann der Schenkel 12 mit einer flachen, am Zahn 7 apikal von dessen Zahnäquator und am angrenzenden Zahnfleisch 15 anliegenden Rippe 13 versehen sein, die zusammen mit einer spiegelbildlichen Rippe 13 am gegenüberliegenden Distanzkörper 3 eine formschlüssige Verrastung der Schiene 2 an den Zähnen des Unterkiefers ermöglicht.

Wie wiederum mit Bezug auf Fig. 1 deutlich wird, enden die oberen Platten 8 der Distanzkörper 3 mesial in Höhe der Eckzähne 4, und das Mittelstück 6 erstreckt sich im Wesentlichen in Verlängerung der lingualen Schenkel 12 der Distanzkörper 3.

Die benachbart zu den Eckzähnen 4 aufragenden Erhebungen 24 sind vorgesehen, um in der Schließstellung des Unterkiefers, d.h. wenn Zähne des Ober- und Unterkiefers an den Distanzkörpern 3 anliegen, zwischen die palatinale Flanke von Eckzähnen des Oberkiefers die mesiale Flanke der ersten Prämolaren des Oberkiefers einzugreifen und dabei beide zu berühren. Die Erhebungen haben jeweils eine distale schräge Flanke 25, die vorgesehen ist, um in der Schließstellung den ersten Prämolar zu berühren, und eine bukkale Flanke 26, die in der Schließstellung gegen den Eckzahn stößt.

Würde man eine zusätzliche Materialschicht auf die distale Flanke 25 auftragen der beiden Distanzkörper 3 aufbringen oder die Erhebungen nach distal verschieben, so hätte dies zur Folge, dass beim Zubeißen der Kontakt der Flanken 25 mit den ersten Prämolaren früher zustande kommt, und bevor die übrigen Backenzähne an die Fläche 10 der Distanzkörper anschlagen, wird durch den Kontakt mit der Flanke 25 der Unterkiefer nach vorn verschoben. Entsprechend ist durch Materialauftrag auf den bukkalen Flanken 26 oder Verschiebung der Erhebungen nach mesial ein früherer Kontakt mit den Eckzähnen des Oberkiefers erreichbar, durch den der Unterkiefer bis zum Erreichen der Schließsstellung rückwärts verschoben wird. In entsprechender Weise ist durch unterschiedlichen Materialauf- oder - abtrag an den bukkalen Flanken 26 beider Distanzkörper oder durch Seitwärtsverschiebung beider Erhebungen in derselben Richtung eine Drehung der Achse des Kiefergelenks erreichbar. So können durch eine passende Formgebung der Erhebungen 24 diverse Kieferfehlstellungen korrigiert und Gelenküberlastungen vermieden werden.

Fig. 3 zeigt einen Querschnitt durch das Mittelstück 6 und einen Schneidezahn 5. Das Mittelstück 6 hat einen stegförmig langgestreckten, beiderseits des Zahnhalses des Schneidezahns 5 an dessen lingualer Flanke 14 sowie am angrenzenden Zahnfleisch 15 anliegenden Querschnitt. Der von dem Mittelstück 6 überdeckte Bereich 16 der lingualen Flanke 14 nimmt hier knapp über die Hälfte von deren Höhe ein; der Rest 17 der Flanke 14, bis hin zur Inzisalkante 18, liegt frei und kann von der Zunge berührt werden. Da die Flanke 14 gegen die Richtung der Kieferbewegung geneigt ist, ist der Schneidezahn 5 in dem Bereich 16 jedes Mal Druck durch das daran anliegende Mittelstück 6 ausgesetzt, wenn Zähne des Oberkiefers auf die Distanzkörper 3 drücken. So bekommt der Zahn 5 regelmäßig den Druckreiz, der erforderlich ist, um eine Verschiebung des Zahns 5 im Kiefer zu vermeiden.

Die Querschnittsfläche der Okklusionsschiene 2 ist im Bereich des Mittelstücks 6 deutlich kleiner als in Höhe der Distanzkörper 3, und auch der in Fig. 3 gezeigte, im Wesentlichen flache Querschnitt des Mittelstücks 6 trägt dazu bei, dass das Mittelstück 6 relativ leicht biegsam ist. Im Falle einer übermäßigen Verbiegung kann es daher auch Schaden nehmen kann, wenn keine geeigneten Gegenmaßnahmen getroffen werden.

Um die Biegebelastbarkeit des Mittelstücks 6 zu vergrößern, kann, wie in Fig. 3 bereits durch einen punktierten Umriss angedeutet, ein metallischer Bügel 19 zur Verstärkung in das Mittelstück 6 eingelegt sein. Um nicht nur Auslenkungen der Distanzkörper 3 quer zur Mesialebene abfedern zu können, sondern auch Torsionsmomente auffangen zu können, hat der Bügel 19 einen unrunden Querschnitt, hier in Form eines flachen Steges, der sich im Wesentlichen parallel zur lingualen Flanke 14 erstreckt und in Richtung dieser Flanke eine erheblich größere Ausdehnung besitzt als senkrecht dazu.

Fig. 4 zeigt eine Okklusionsschiene 2 mit dem darin eingebetteten Bügel 19. Der Bügel 19 reicht auf beiden Seiten weit über das Mittelstück 6 hinaus, und hat distale Bereiche 20, die tief in die lingualen Schenkel 12 der Distanzkörper 3 hineinreichen. Um die Toleranz gegen Torsionsbelastung zu steigern, ist in den distalen Bereichen 20 das Aspektverhältnis zwischen den Querschnittabmessungen des Bügels 19 in Apikal/Zervikal-Richtung d bzw. senkrecht zur angrenzenden Zahnoberfläche b größer als im Mittelstück 6. Um zu verhindern, dass wenn zwischen dem Kunststoff des Distanzkörpers 3 und dem Bügel 19 Torsionskräfte auftreten, diese den Kunststoff der Distanzkörper 3 sprengen, ist der Bügel 19 dort, wo er seine maximale Ausdehnung d in Apikal/Zervikal-Richtung erreicht, gegabelt, so dass der Kunststoff an Innen- und Außenseite des Bügels 19 über einen Zwischenraum 22 zwischen den Zinken 21 der Gabel materialschlüssig zusammenhängt, wie insbesondere aus Fig. 2 deutlich wird.

Fig. 5 zeigt eine Ausgestaltung der Okklusionsschiene, bei der das Mittelstück 6 allein durch den metallischen Bügel 19 gebildet ist und außer diesem Bügel 19 keine weitere Verbindung zwischen den Distanzkörpern 3 existiert. Die Verankerung der distalen Bereiche 20 des Bügels 6 in den Distanzkörpern ist dieselbe wie mit Bezug auf Fig. 2 und 3 beschrieben.

Der metallische Bügel 19 kann ersetzt werden durch eine Einlage aus Fasermaterial, insbesondere Kohlenstoff-, Glas- oder Aramidfasern. Die Einlage kann in ein Matrixmaterial eingebettet und zu einem Bügel mit dem metallischen Bügel 19 entsprechender Gestalt vorgeformt sein, an den anschließend die Distanzkörper 3 angeformt werden. Um in einem solchen Fall einen festen Zusammenhalt zwischen Einlage und Distanzkörpern 3 zu gewährleisten, ist das Material der Distanzkörper 3 vorzugsweise mit dem die Fasereinlage aufnehmenden Matrixmaterial chemisch identisch oder zumindest in soweit chemisch kompatibel, dass beide eine innige materialschlüssige Verbindung eingehen können. Denkbar ist aber auch, die Schiene 2 vollständig aus faserverstärktem Kunststoff zu fertigen, z.B. durch Einspritzen von Matrixmaterial mit darin suspendierten kurzen Fasern in eine Hohlform, durch Einspritzen von Matrixmaterial in eine mit Fasern ausgelegte Hohlform, oder durch Einlegen von mit Matrixmaterial ummantelten Fasern in die Hohlform und anschließend Erhitzen, um die Matrixmäntel der Fasern miteinander zu verschmelzen. Insbesondere letztere Techniken eignen sich zur Fertigung einer hoch belastbaren Okklusionsschiene 2, da die Fasern so verlegt werden können, dass sie sich bei der fertigen Okklusionsschiene 2 in Längsrichtung des Mittelstücks 6 erstrecken.

Fig. 6 zeigt eine Variante der Okklusionsschiene 2, in der sich Fasern 23 der Einlage überwiegend durchgehend von einem Distanzkörper 3 in den anderen erstrecken. Die Dichte der Fasern 23 ist dabei im Mittelstück 6 am größten, da dieses eine kleinere Querschnittsfläche als die Distanzkörper 3 hat, wodurch gleichzeitig aber auch die Zähigkeit des Mittelstücks 6 in durchaus erwünschter Weise größer wird als die der Distanzkörper 3. Beim Eintritt in die Distanzkörper 3 laufen die Fasern 23 auseinander, so dass die Platte 8 und Schenkel 11, 12 des Distanzkörpers im Querschnitt im Wesentlichen gleichmäßig von Fasern 23 durchsetzt sind.

### Bezugszeichen

- 1: Abguss
- 2: Okklusionsschiene
- 3: Distanzkörper
- 4: Eckzahn
- 5: Schneidezahn
- 6: Mittelstück
- 7: Backenzahn
- 8: obere Platte
- 9: Einbuchtung
- 10: Fläche
- 11: bukkaler Schenkel
- 12: lingualer Schenkel
- 13: Rippe
- 14: linguale Flanke
- 15: Zahnfleisch
- 16: Bereich
- 17: Rest
- 18: Inzisalkante
- 19: Bügel
- 20: distaler Bereich
- 21: Zinken
- 22: Zwischenraum
- 23: Faser
- 24: Erhebung
- 25: Flanke
- 26: Flanke

## Patentansprüche

1. Okklusionsschiene zur Anbringung am Unterkiefer eines Patienten, mit einem linken und einem rechten Distanzkörper (3), die geformt sind, um wenigstens einen linken bzw. einen rechten Backenzahn (7) des Unterkiefers zu überdecken, und einem sich bogenförmig zwischen dem linken und dem rechten Distanzkörper (3) erstreckenden Mittelstück (6), **dadurch gekennzeichnet, dass** das Mittelstück (6) Schneidezähne (5) des Patienten lingual berührt und deren Inzisalkante (18) unbedeckt lässt.

2. Okklusionsschiene nach Anspruch 1, **dadurch gekennzeichnet, dass** das Mittelstück (6) ferner die lingualen Flanken (14) der Schneidezähne (5) in einem an die Inzisalkante (18) angrenzenden Bereich (17) unbedeckt lässt.

3. Okklusionsschiene nach Anspruch 2, **dadurch gekennzeichnet, dass** der unbedeckte Bereich (17) der lingualen Flanken (14) wenigstens ein Viertel, vorzugsweise wenigstens ein Drittel der Flanken (14) einnimmt.

4. Okklusionsschiene nach einem der vorhergehenden Ansprüche, **dadurch gekennzeichnet, dass** das Mittelstück (6) an die Schneidezähne (5) angrenzendes Zahnfleisch (15) überdeckt.

5. Okklusionsschiene nach einem der vorhergehenden Ansprüche, **gekennzeichnet durch** einen Bügel (19), von dem distale Bereiche (20) formschlüssig in den Distanzkörpern (3) eingebettet sind und ein zentraler Bereich Teil des Mittelstücks (6) ist.

6. Okklusionsschiene nach Anspruch 5, **dadurch gekennzeichnet, dass** der Bügel (19) aus Metall und die Distanzkörper (3) aus einem Kunstharz geformt sind.

7. Okklusionsschiene nach Anspruch 5 oder 6, **dadurch gekennzeichnet, dass** die distalen Bereiche (20) ein höheres Querschnitts-Aspektverhältnis aufweisen als der zentrale Bereich des Bügels (19).

8. Okklusionsschiene nach Anspruch 7, **dadurch gekennzeichnet, dass** die distalen Bereiche (20) jeweils zwei Zweige (21) umfassen, die durch einen mit Material der Distanzkörper (3) ausgefüllten Zwischenraum (22) getrennt sind.

9. Okklusionsschiene nach einem der Ansprüche 5 bis 8, **dadurch gekennzeichnet, dass** der linke und der rechte Distanzkörper (3) nur durch den Bügel (19) verbunden sind.

10. Okklusionsschiene nach einem der vorhergehenden Ansprüche, dadurch nach einem der vorhergehenden Ansprüche, **dadurch gekennzeichnet, dass** wenigstens ein Teil des Mittelstücks (6) mit dem linken und dem rechten Distanzkörper (3) einteilig zusammenhängt.

11. Okklusionsschiene nach Anspruch 10, soweit auf Anspruch 5 rückbezogen, **dadurch gekennzeichnet, dass** der Bügel (19) beabstandet von den Schneidezähnen (5) verläuft.

12. Okklusionsschiene nach einem der vorhergehenden Ansprüche, **dadurch gekennzeichnet, dass** wenigstens das Mittelstück (6) eine Einlage aus Fasern (23), insbesondere Glas-, Kohlenstoff- oder Aramidfasern aufweist.

13. Okklusionsschiene nach Anspruch 12, **dadurch gekennzeichnet, dass** eine Matrix, in die die Fasern (23) eingebettet sind, und ein faserfreier Bereich der Distanzkörper (3) aus gleichen oder zumindest kompatiblen Kunststoffen bestehen.

14. Okklusionsschiene nach einem der vorhergehenden Ansprüche, **dadurch gekennzeichnet, dass** die Distanzkörper (3) an ihrem vorderen Ende eine Erhebung (24) tragen.

15. Okklusionsschiene nach Anspruch 14, **dadurch gekennzeichnet, dass** die Erhebung (24) eine erste Flanke (26) aufweist, die in Schließstellung des Unterkiefers die palatale Flanke eines Eckzahns des Oberkiefers berührt, und eine zweite Flanke (25) aufweist, die in der Schließstellung die mesiale Flanke eines ersten Prämolars des Oberkiefers berührt.
